(19) Europäisches Patentamt
European Patent Office
Office européen des brevets

(11) Veröffentlichungsnummer: **0 033 439**
**B1**

# (12) EUROPÄISCHE PATENTSCHRIFT

(45) Veröffentlichungstag der Patentschrift: **29.05.85**

(51) Int. Cl.[4]: **C 12 P 33/00**, C 07 J 13/00

(21) Anmeldenummer: **81100146.0**

(22) Anmeldetag: **10.01.81**

## (54) Neue delta-1,4,17-BNC-Verbindungen und Verfahren zu ihrer Herstellung.

(30) Priorität: **04.02.80 AT 582/80**

(43) Veröffentlichungstag der Anmeldung: **12.08.81 Patentblatt 81/32**

(45) Bekanntmachung des Hinweises auf die Patenterteilung: **29.05.85 Patentblatt 85/22**

(84) Benannte Vertragsstaaten: **BE CH DE FR LI NL**

(56) Entgegenhaltungen:
**EP - A - 0 004 913**
**US - A - 3 994 933**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Bahn, Michael, Dr., Frans-Hals-Weg 19, D-4010 Hilden/Rhld. (DE)**
Erfinder: **Schmid, Rolf, Dr., Am Nettchesfeld 30, D-4000 Düsseldorf 13 (DE)**
Erfinder: **Wagner, Rüdiger, Dr., Haferbuckel 27, D-4019 Monheim/Rhld. (DE)**

**EP 0 033 439 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Gegenstand der offengelegten europäischen Patentanmeldung 0 004 913 ist unter anderem ein Verfahren zur Herstellung von 17-C-Steroid-$\alpha$-propionsäureverbindungen — insbesondere zur Herstellung von 3-Oxo-pregna-4-en-20-carbonsäure ($\Delta$4-BNC) und/oder 3-Oxo-pregna-1,4-dien-20-carbonsäure ($\Delta$1,4-BNC) — durch mikrobiellen Seitenkettenabbau an 17-C-Seitenketten-Steroidsubstraten. Das Verfahren ist dadurch gekennzeichnet, daß man Mikroorganismen-Defektmutanten, die auch in Abwesenheit von den Steroidringabbau und/oder das Wachstum hemmenden Inhibitoren Steroidverbindungen mit dem 17-C-$\alpha$-Propionsäurerest liefern, in einem wäßrigen Nährmedium unter aeroben Bedingungen in Gegenwart des Steroidsubstrats unter Anreicherung der 17-C-Steroid-$\alpha$-propionsäureverbindungen in der Fermentationsbrühe züchtet und die gebildete $\Delta$4-BNC und/oder $\Delta$1,4-BNC isoliert.

Vorzugsweise wird mit Defektmutanten als Mikroorganismen gearbeitet, die durch Mutation und anschließende Selektion aus solchen zuvor ausgewählten Wildstämmen gezüchtet worden sind, die auf Steroidverbindungen mit 17-C-Seitenketten als vorzugsweise einziger Kohlenstoffquelle mit wenigstens etwa gleicher Abbaugeschwindigkeit für die Seitenketten wie für den Ringanteil der Steroidverbindungen zu wachsen vermögen, vorzugsweise aber eine erhöhte Seitenkettenabbaugeschwindigkeit besitzen. Insbesondere sind dabei die eingesetzten Blockmutanten aus Wildstämmen gezüchtet worden, die beim Wachstum auf Steroidverbindungen eine selektive Abbauleistung unter Standardbedingungen gemäß der allgemeinen Formel $I = a \cdot 10^b$ ergeben, worin a der Wachstumsfaktor und b der Selektivitätsfaktor des Wildstammwachstums sind und der Selektivitätsindex I den Zahlenwert von wenigstens 10, vorzugsweise von wenigstens 100 aufweist. Insbesondere beträgt der Selektivitätsindex I des Wildstamms wenigstens $10^5$. Der Selektivitätsfaktor b des zur Züchtung der Defektmutanten eingesetzten Wildstammes soll vorzugsweise wenigstens 2 und der Wachstumsfaktor a vorzugsweise wenigstens 0,2, insbesondere wenigstens 1 betragen.

Die Züchtung der Wildstämme zur anschließenden Selektion gemäß ihres Selektivitätsindex I wird dabei auf einer 17-C-Seitenketten-Steroidverbindung derjenigen Art als Kohlenstoffquelle durchgeführt, die im Abbauverfahren als Einsatzmaterial zur Verwendung kommt, wobei vorzugsweise diese Steroidverbindung als einzige Kohlenstoffquelle zur Züchtung der Wildstämme eingesetzt wird.

Als Defektmutanten werden vorzugsweise solche Stämme eingesetzt, die bei der Züchtung der Mutantenpopulation aus einer an sich bekannten Mutation der ausgewählten Wildstämme auf einem Mutanten-Trennmedium nicht oder praktisch nicht wachsen, während die unerwünschten begleitenden Mutantenstämme wachsen und hierdurch bzw. während ihres Wachstums abgetötet worden sind. Vorzugsweise wird mit Blockmutanten von Achromobacter, Arthrobacter, Bacillus, Brevibacterium, Corynebacterium, Flavobacterium, Microbacterium, Mycobacterium, Nocardia, Protaminobacterium, Serratia oder Streptomyces gearbeitet.

Als Steroidausgangsverbindungen werden solche mit gesättigten und/oder ungesättigten 17-C-Seitenketten eingesetzt, wobei vorzugsweise diese Seitenkettenreste bis zu 10 C-Atome, insbesondere 8 bis 10 C-Atome, aufweisen. Besonders geeignet als Ausgangsmaterialien sind Sterine tierischen und/oder pflanzlichen Ursprungs, insbesondere Cholesterin, Sitosterin, Stigmasterin, Campesterin und/oder Ergosterin oder ihre Abkömmlinge, wie Cholestenon, Sitostenon oder Stigmastenon.

Zu weiteren Einzelheiten wird auf die genannte veröffentlichte europäische Patentanmeldung 0 004 913 verwiesen.

Eine weitere Ausgestaltung dieses Verfahrens zur Herstellung von $\Delta$4-BNC und $\Delta$1,4-BNC ist in der offengelegten europäischen Patentanmeldung 0 015 308 beschrieben. Hier wird mit Defektmutanten gearbeitet, die in analoger Weise jedoch aus einem solchen Wildstamm gewonnen worden sind, der bei aeroben Wachstum auf Sterinverbindungen in Gegenwart von Inhibitoren für den enzymatischen Ringabbau der Sterinverbindungen wenigstens anteilsweise 17-C-Steroid-$\alpha$-propionsäureverbindungen liefert. Insbesondere werden dort zunächst solche Wildstämme isoliert und gezüchtet, die beim Wachstum auf Sterinverbindungen mit gesättigten oder ungesättigten Alkylresten mit 17-C- mit 8 bis 10 C-Atomen eine Ausbeute von 17-C-Steroid-$\alpha$-propionsäureverbindungen — gemessen unter Standardbedingungen — von wenigstens 5 Gew.-%, vorzugsweise von wenigstens 10 Gew.-% bezogen auf die eingesetzten Sterinverbindungen — liefern.

Vorzugsweise werden solche Mikroorganismenwildstämme isoliert und gezüchtet, die beim Wachstum auf Sterinverbindungen der genannten Art eine selektive Abbauleistung gemäß der allgemeinen Formel $I = a \cdot 10^p$ ergeben, worin a der Wachstumsfaktor und p der Selektivitätsfaktor des Wildstammwachstums sind und der Selektivitätsindex I den Zahlenwert von wenigstens 1, vorzugsweise von wenigstens 2 und insbesondere den Wert von wenigstens 20 beträgt. Der Wachstumsfaktor a des Wildstammes soll unter Standardbedingungen wenigstens 0,2, vorzugsweise wenigstens 1 und der Selektivitätsfaktor p unter Standardbedingungen wenigtens 0,5, vorzugsweise wenigstens 1, betragen. Die bevorzugten Wildstämme zur anschließenden Gewinnung der Defektmutanten sollen den 17-C-Seitenkettenabbau einem Ringabbau gegenüber bevorzugen. Die Mutationsbehandlung der Wildstämme erfolgt unter solchen Bedingungen von Konzentration und Einwirkzeit des mutagenen Agenz, daß die Ausgangspopulation der Mikroorganismen durch die mutagene Behandlung zu 10 bis 99,999% inaktiviert wird, wobei vorzugsweise mit einer Abtötungsrate von 90 bis 99,99% gearbeitet wird.

Es wurde jetzt gefunden, daß bei der Fermentation von Sterinverbindungen mit Mikroorganismen-Defektmutanten gemäß der Lehre der europäischen Patentanmeldungen 0 004 913 und 0 015 308 auch das bisher nicht beschriebene Abbauprodukt 20-Carboxy-pregna-1,4,17(20)-trien-3-on — im folgenden als Δ1,4,17-BNC bezeichnet — gewonnen werden kann.

Die Strukturformeln der Δ1,4,17-BNC ist die folgende:

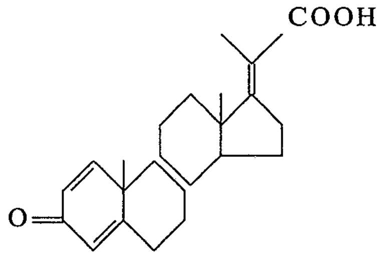

(II)

Gegenstand der vorliegenden Erfindung ist dementsprechend in einer ersten Ausführungsform die neue Verbindung Δ1,4,17-BNC sowie der entsprechende Methylester dieser neuen Verbindung. Dieses Derivat ist ebenfalls bisher nicht beschrieben. Die neuen Verbindungen der Erfindung entsprechen der allgemeinen Formel

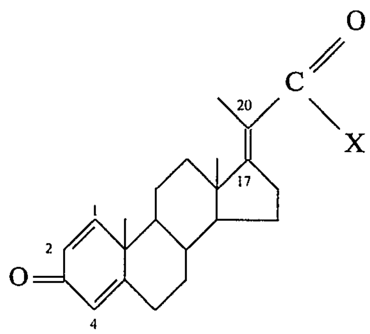

(I)

In dieser Formel bedeutet X den Rest OH oder $OCH_3$. Der Methylester kann aus der Δ1,4,17-BNC gewonnen werden. Einzelheiten hierzu werden im folgenden angegeben.

Die Erfindung betrifft weiterhin ein Verfahren zur Herstellung von 20-Carboxy-pregna-1,4,17(20)-trien-3-on (Δ1,4,17-BNC) durch mikrobiellen Seitenkettenabbau an 17-C-Seitenketten-Steroidsubstraten in einem wäßrigen Nährmedium unter aeroben Bedingungen mit Mikroorganismen-Defektmutanten, die auch in Abwesenheit von den Steroidringabbau und/oder das Wachstum hemmenden Inhibitoren Steroidverbindungen mit dem 17-C-α-Propionsäurerest liefern und die gemäß den europäischen Patentanmeldungen 0 004 913 und 0 015 308 erhältlich sind, dadurch gekennzeichnet, daß man die Mikroorganismen-Defektmutanten bei gedrosselter Luftzufuhr unter Anreicherung der Δ1,4,17-BNC züchtet und diese aus dem Reaktionsprodukt abtrennt.

Zu den Einzelheiten der Gewinnung der Mikroorganismen-Defektmutanten gelten die Angaben der veröffentlichten europäischen Patentanmeldungen 0 004 913 sowie 0 015 308. In weiterer Ausbildung der Verfahren zum Sterinabbau mit diesen Defektmutanten wurde jetzt gefunden, daß durch Drosselung der Luftzufuhr während der Fermentation die bisher nicht beschriebene Δ1,4,17-BNC in derart erhöhten Ausbeuten gewonnen werden kann, daß ihre technische Gewinnung interessant wird.

Durch Variation des Sauerstoffunterschusses in der Fermenterflüssigkeit kann Einfluß auf die Ausbeute an der gewünschten Δ1,4,17-BNC genommen werden, wobei die jeweils optimalen Arbeitsbedingungen bezüglich des Sauerstoffunterschusses stammabhängig sind und durch einfache Vorversuche von Fall zu Fall ermittelt werden können. Allgemein gilt, daß bevorzugt mit einer Sättigungskonzentration des Sauerstoffs in der Fermenterflüssigphase von höchstens etwa 90% $pO^2$ gearbeitet wird, wobei 100% $pO^2$ der Partialdruck des in der Fermenterflüssigphase unter Arbeitsbedingungen gelösten Sauerstoffs bei unbeschränkter Zufuhr von Luft bzw. Sauerstoff ist. Es kann erfindungsgemäß weiterhin bevorzugt sein, mit entsprechenden Sättigungskonzentrationen des Sauerstoffs in der Fermenterflüssigphase im Bereich von 30 bis 70% $pO^2$ zu arbeiten. Die Einstellung und Aufrechterhaltung vorbestimmter Sättigungskonzentrationen durch Drosselung der Zufuhr von Sauerstoff ist im erfindungsgemäßen Verfahren ohne besondere Schwierigkeiten möglich. Die erfindungsgemäß eingesetzten Mikroorganismen-Defektmutanten sind vergleichsweise langsam wachsend, was die Steuerung des in der Fermenterflüssigphase gelösten Sauerstoffs erleichtert.

Bei einer Reihe von erfindungsgemäß eingesetzten Mikroorganismen-Defektmutanten führt die Drosselung der Luftzufuhr zu einer beschränkten Senkung der Gesamtausbeute an BNC-artigen Verbindungen gegenüber einem Verfahren mit unbeschränktem Luftzutritt. Als BNC-artige Verbindungen, die beim Arbeiten mit den hier betroffenen Mutantenstämmen entstehen bzw. entstehen können, sind

in erster Linie die in den genannten europäischen Patentanmeldungen beschriebene Δ4-BNC und die Δ1,4-BNC sowie in untergeordnetem Maße auch die Δ4,17(20)-BNC zu nennen. Beim Arbeiten mit Mutantenstämmen, bei denen die Drosselung der Luftzufuhr zu einer Senkung der Gesamtausbeute führt, kann es bevorzugt sein, das Ausmaß der Ausbeutesenkung auf wenigstens 5%, vorzugsweise auf wenigstens 10% — verglichen mit der Standardausbeute bei unbeschränkter Luftzufuhr — festzulegen. Es kann sogar zweckmäßig sein, mit noch stärkeren Einschränkungen der Ausbeute zu arbeiten, so daß — jeweils pro Zeiteinheit und unter sonst vergleichbaren Bedingungen — die Senkung der Gesamtausbeute an BNC-artigen Verbindungen 30%, 40% oder gar 50% beträgt. Hierbei steigt die Ausbeute an Δ1,4,17-BNC, bezogen auf das jeweils gewonnene gesamte Reaktionsprodukt.

Es wurde weiterhin gefunden, daß eine Reihe von ausgewählten Mikroorganismen-Defektmutanten in der Lage ist, unabhängig von der Regulierung der Luftzufuhr erhöhte Mengen an Δ1,4,17-BNC zu bilden. Auch hier gilt allerdings, daß durch Drosselung der Luftzufuhr während der Fermentation die Ausbeute an gewünschtem Verfahrensprodukt weiter erhöht werden kann. Geeignet sind insbesondere Tochterstämme des bei der Deutschen Sammlung für Mikroorganismen (DSM) in Göttingen hinterlegten Stammes mit der internen Bezeichnung T 191, Hinterlegungsnummer DSM 1444. Die Hinterlegungsnummern der im Rahmen der Erfindung bevorzugt eingesetzten Mikroorganismen-Defektmutanten sind die folgenden: DSM 1990 (Stamm SC-372-837) und ATCC 31 636 (Stamm T 191-1091).

Es hat sich weiterhin als zweckmäßig erwiesen zur Ausbeutesteigerung an Δ1,4,17-BNC im Fermenter wenigstens teilweise wassermischbare Lösungsmittel für das Steroidausgangssubstrat einzusetzen. Insbesondere kann es dabei zweckmäßig sein, solche Lösungsmittel mit zu verwenden, die unbeschränkt wassermischbar sind. Dabei wird vorzugsweise das Steroidsubstrat im wassermischbaren Lösungsmittel gelöst und in dieser Form in den Fermenter eingetragen. Als Lösungsmittel der hier betroffenen Art kommen organische Lösungsmittel in Betracht. Beispiele sind entsprechende Alkohole, insbesondere niedere Alkohole wie Methanol oder Ethanol, Dimethylformamid, Dioxan, Tetrahydrofuran oder Dimethylsulfoxid. Die Transformation des eingesetzten Sterinsubstrats zur Δ1,4,17-BNC kann auf diese Weise nicht nur mit verbesserten Ausbeuten, sondern zusätzlich auch mit beträchtlicher Reaktionsbeschleunigung erfolgen.

Die wassermischbaren organischen Lösungsmittel werden vorzugsweise in einer mehrfachen Gewichtsmenge, bezogen auf eingesetztes Steroidsubstrat, verwendet. Bevorzugt sind Mischungsverhältnisse von Steroidsubstrat/Lösungsmitteln im Bereich von 1 Gewichtsteil : 5—100 Gewichtsteile. Insbesondere ist es bevorzugt mit Mischungsverhältnissen von 1 : 10 bis 30 Gewichtsteilen zu arbeiten.

Zu Einzelheiten des Züchtungsverfahrens gelten die Angaben der offengelegten europäischen Patentanmeldung 0 004 913. So sind als Steroidausgangsmaterialien insbesondere solche mit einer gesättigten und/oder ungesättigten 17-C-Seitenkette mit vorzugsweise bis zu 10 C-Atomen, insbesondere mit 8 bis 10 C-Atomen geeignet. Bevorzugt werden Sterine tierischen und/oder pflanzlichen Ursprungs als Ausgangsmaterial eingesetzt. Besonders geeignete Ausgangsmaterialien sind Cholesterin, Sitosterin, Stigmasterin, Campesterin und/oder Ergosterin sowie ihre Abkömmlinge wie Cholestenon, Sitostenon oder Stigmastenon.

Die als Einsatzmaterial gewählte Steroidverbindung kann der Kultur während der Inkubationsperiode zugesetzt werden oder sie kann dem Nährmedium vor der Inokulierung der Defektmutanten beigegeben werden. Es kann eine Steroidverbindung oder auch eine Mischung von mehreren Steroidverbindungen eingesetzt werden. Vorzugsweise werden die selektiv abzubauenden Steroidverbindungen in der Kultur in Mengen von etwa 0,1 bis 100 g/l verwendet. Die optimale Konzentration der zu transformierenden Sterinverbindung in der Züchtungsstufe ist im allgemeinen stammabhängig und kann durch einfache Vorversuche jeweils ermittelt werden. Im allgemeinen liegt die Konzentration der Sterinverbindung im Medium vorzugsweise nicht über 30 g/l und häufig nicht über 20 g/l.

Es kann dabei bevorzugt sein, das dem Seitenkettenabbau zu unterwerfende Substrat nicht auf einmal dem Reaktionsmedium zuzuetzen, sondern diese Zugabe nach und nach im Verlauf der Reaktion vorzunehmen. Vorzugsweise wird das Ausgangssubstrat in dieser Ausführungsform im wesentlichen kontinuierlich im Reaktionsgemisch im Verlauf der Abbaureaktion zugegeben. Auf diese Weise kann häufig die Ausbeute der gewünschten Abbauprodukte erhöht werden.

Die Kultur wird in einem Nährmedium gezüchtet, das als Kohlenstoffquelle entweder die zu transformierenden Sterine oder aber auch noch zusätzliche metabolisierbare Kohlenstoffquellen sowie die üblicherweise von diesen Mikroorganismen benötigten Nähr- und Wuchsstoffe enthält. Besonders günstig für das Wachstum der Mikroorganismen sind z. B. Paraffin, Glycerin, Carbonsäuren, Stärke, Dextrin, Saccharose, Glucose, Fructose und zuckerhaltige Abfallstoffe. Geeignete Stickstoffquellen sind Ammoniumsalze, Nitrate, Pepton, Maisquellwasser, Maiskleber, Sojamehl, Schlempe und Fischmehl. Weiterhin können Fermentationsbeschleuniger, wie Hefeextrakt und Vitamine zugesetzt werden. Das Nährmedium enthält darüber hinaus zweckmäßigerweise anorganische Salze, wie Natrium-, Kalium- oder Ammoniumphosphate sowie Calcium-, Magnesium-, Mangan- oder Eisensalze. Die Emulgierung der Sterine in dem Nährmedium erfolgt vorzugsweise mittels bekannter Emulgatoren, z. B. mittels Fettsäure-Sorbitanester oder deren Ethylenoxidaddukten, Polyoxyethylenmonolaurylether oder Fettsäureamidalkylbetain.

Das verwendete Kulturmedium wird vor Beginn der Bakterienanzucht zweckmäßigerweise durch Erhitzen sterilisiert. Nach dem Abkühlen und Beimpfen des Kulturmediums mit einer geeigneten

Vorkultur des transformierenden Bakterienstammes wird zwischen 25 bis 55°C inkubiert, vorzugsweise bei 27 bis 30°C. Der pH-Wert der Nährlösung liegt zwischen pH 4,4 bis 8,5, vorzugsweise bei 7,0 bis 8,0. Die Kultur wird durch Schütteln, Rühren oder Gaseinleiten mit Sauerstoff im gewünschten Ausmaß versorgt und bis zum Abbau des Sterins bis zur gewünschten Stufe inkubiert. Der Abbau des Sterins fordert je nach Substratkonzentration und den anderen Fermentationsbedingungen in der Regel 24 bis 160 Stunden. Es hat sich dabei gezeigt, daß die gewünschte $\Delta$1,4,17-BNC häufig rascher das angestrebte Ausbeuteoptimum erreicht als andere begleitende BNC-Verbindungen. Insoweit kann es erfindungsgemäß zweckmäßig sein, nicht auf eine maximale Gesamtumsetzung des Sterinausgangsmaterials abzustellen, sondern — unter Verkürzung der Abbauperiode — das gebildete Verfahrensprodukt dann zu isolieren, wenn der gewünschte Ausbeutegrad an $\Delta$1,4,17-BNC erreicht ist.

Das auf diese Weise hergestellte Verfahrensprodukt, das sich üblicherweise in der Fermentationsbrühe anreichert, kann dann in an sich bekannter Weise aus dem Reaktionsgemisch gewonnen werden. So können beispielsweise BNC-Verbindungen durch Extraktion mit organischen Lösungsmitteln, wie Methylenchlorid, Methylisobutylketon, Essigsäureester, n-Hexanol, n-Octanol, Chloroform oder n-Hexan, aus dem Kulturmedium vor oder nach Abtrennen der Zellen isoliert werden.

Nach einer bevorzugten Ausführungsform gelingt die Isolierung des Reaktionsproduktes aus der Fermenterflüssigkeit ganz einfach durch Ausfällung im sauren Bereich und Abfiltrieren. Hierzu wird die alkalisch eingestellte Fermenterflüssigkeit zunächst filtriert, um das Zellmaterial und andere feste Bestandteile zu entfernen. Anschließend wird angesäuert. Das in fester filtrierbarer Form ausgefällte Produkt kann z. B. durch einfaches Abnutschen gewonnen werden. Das Reaktionsprodukt wird anschließend aufgetrennt und hierdurch die $\Delta$1,4,17-BNC in reiner Form gewonnen. Der ausgefallene Feststoff besteht fast vollständig aus einer Mischung von $\Delta$1,4,17-BNC, $\Delta$1,4-BNC (20-Carboxy-pregna-1,4-dien-3-on) und $\Delta$4-BNC (20-Carboxy-pregna-4-en-3-on). Die Auftrennung gelingt z. B. durch chromatographische Methoden in Form der freien Säure oder nach Veresterung in Form des Methylesters.

Die Umwandlung der freien Säure in den Methylester ist in an sich bekannter Weise durch Reaktion mit Diazomethan möglich. Die Umsetzung selbst erfolgt in literaturbekannter Weise.

$\Delta$1,4,17-BNC ist ein wichtiges Zwischenprodukt für die Partialsynthese von pharmazeutisch wirksamen Steroiden. Ein besonderer Vorteil gegenüber $\Delta$1,4- bzw. $\Delta$4-BNC liegt in dem Vorliegen der zusätzlichen 17(20)-Doppelbindung, die eine häufig erwünschte Einführung eines Substituenten am C-Atom 17 ermöglicht bzw. erleichtert.

Die US-PS 3 994 933 beschreibt 20-Carboxy-pregna-4,17(20)-dien-3-on und dessen Herstellung durch mikrobiellen Abbau aus Sterinen. Diese bekannte Verbindung unterscheidet sich von der erfindungsgemäßen Verbindung durch das Fehlen der $\Delta$1-Doppelbindung. Die Anwesenheit dieser Doppelbindung kann jedoch bei der Verwendung als Pharma-Zwischenprodukt ein wichtiger Vorteil gegenüber der bekannten Verbindung sein.

In den folgenden Beispielen sind Prozentangaben jeweils Gewichtsprozent soweit nicht etwas anderes angegeben ist.

## Beispiel 1

Die im folgenden angegebenen Mikroorganismen-Defektmutanten werden in 500 ml Erlenmeyerkolben mit 100 ml Nährlösung folgender Zusammensetzung aerob gezüchtet: 0,5% Pepton, 0,8% Hefeextrakt, 0,4% Maiskleber, 0,3% Glukose, 0,05% Tween 80 (Polyoxyäthylensorbitanmonooleat), 0,05% Cholesterin, pH 7,2. Die Kultur wurde auf der Schüttelmaschine (Schüttelfrequenz 150 UpM) bei 30°C für 48 Stunden vorgezüchtet, anschließend 0,2% Emulgator und 0,1% Cholesterin zugegeben und für weitere 120 Stunden bebrütet. Nach Abbruch der Kulturen wurden Proben genommen, auf pH 2,0 eingestellt, 1 : 1 mit Essigester extrahiert und dünnschichtchromatographisch analysiert. Die Ausbeuten der einzelnen Stämme sind in der nachfolgenden Tabelle angegeben.

Um bei diesen Versuchsarbeiten geringere Luftsättigungswerte im Reaktionsmedium zu erzeugen, wurden anstelle der aus dem Stand der Technik bekannten Schüttelkolben mit jeweils vier Schikanen (=Einkerbungen der Glaswand in Bodennähe von ca. 2 m Länge und 1 cm Tiefe) erfindungsgemäß auch unter Verwendung schikanenfreier Schüttelkolben gearbeitet.

Die Identifizierung der Verbindungen erfolgte durch High-Performance-Dünnschichtchromatographie.

Die Ergebnisse bei den verschiedenen Arbeitsweisen sind wie folgt:

Tabelle

| Interne Nummer | Hinterlegungs-nummer | Bedingungen | $\Delta^{1,4}$-BNC mg/100 ml | $\Delta^{1,4,17}$-BNC mg/100 ml |
|---|---|---|---|---|
| T 191 | DSM 1444 | gemäß Beispiel 3 der veröffentlichten europ. Patentanmeldung 0 004 913 | 80 | 12 |
| T 191-1091 | ATCC 31 636 | wie oben | 95 | 19 |
| | | wie oben, aber mit schikanenfreien Kolben | 34 | 10 |

Beispiel 2

Die freie $\Delta^{1,4,17}$-BNC wird nach ihrer Isolierung aus dem Reaktionsgemisch mit Diazomethan in ihren Methylester übergeführt und in dieser Form einer Feinreinigung durch präparative Hochdruck-Flüssigchromatographie unterworfen. Die Bedingungen hierfür sowie die spektroskopischen Daten des auf diese Weise gereinigten Methylesters sind im folgenden angegeben:

Bedingungen für die Hochdruck-Flüssigchromatographie

Säule: Kieselgelsäule der Firma Du Pont, gefüllt mit Zorbox SIL, mittlere Teilchengröße 7 µ.

| | |
|---|---|
| Länge: | 250 mm |
| Innendurchmesser: | 23 mm |
| Elutionsmittel: | Isooctan/Isopropanol (98 : 2) |
| Bedingungen: | 50 ml/Min. Durchfluß |
| | 50 bar |
| | 298 k |
| Detektion: | UV-Detektor, 254 nm. |

Spektroskopische Daten

1. IR-Spektrum ($CHCl_3$): 
   - 1714 $cm^{-1}$ (C=O, Ester)
   - 1660 $cm^{-1}$ (C=O, Keton)
   - 1625 $cm^{-1}$
   - 1603 $cm^{-1}$

2. $^1H$—NMR-Spektrum (80 MHz, $CDCl_3$, $\delta$-Werte):

| | |
|---|---|
| 18-$CH_3$: | 0,99 ppm, s |
| 19-$CH_3$: | 1,24 ppm, s |
| 21-$CH_3$: | 1,93 ppm, t (J = 1,9 Hz) |
| O—$CH_3$: | 3,69 ppm, s |
| olefinische Protonen: | ABC-System mit Linien bei 6,07; 6,12; 6,15; 6,27; 6,30; 6,97; 7,10 ppm. |

die restlichen Protonen (Methylen- und Methinprotonen) liefern Signale im erwarteten Bereich.

3. $^{13}C$-NMR, breitbandrauschentkoppelt und Off Resonance, (20 MHz, $CDCl_3$, TMS als Standard)

| | |
|---|---|
| C-18, C-19, C-21: | 14,6 ppm, q |
| | 15,5 ppm, q |
| | 18,7 ppm, q |
| O—$CH_3$: | 51,1 ppm, q |
| C-1: | 155,5 ppm, d |
| C-2: | 127,7 ppm, d |

| | |
|---|---|
| C-3: | 186,2 ppm, s |
| C-4: | 124,0 ppm, d |
| C-5: | 168,6 ppm, s |
| C-22: | 162,8 ppm, s |
| C-17: | 155,3 ppm, s |
| C-20: | 118,6 ppm, s |
| Weitere Signale bei: | 23,1; 24,8; 32,7; 33,5; 35,0; 36,5; 43,4; 46,9; 51,9; 54,6; 55,1 ppm |

4. UV-Spektrum:
   $\lambda$max (Isopropanol): 239 nm

5. Massen-Spektrum: M+ 354 µm

Weitere Angaben

Schmelzpunkt: 155 bis 161°C.

## Beispiel 3

Die Versuche des Beispiels 1 werden unter den dort angegebenen Bedingungen wiederholt, es wird jedoch als Steroidsubstrat anstelle von Cholesterin ein aus Sojabohnenöl gewonnenes Gemisch pflanzlicher Sterinverbindungen eingesetzt. Die Zusammensetzung dieses Pflanzensterins ist wie folgt: Gesamtgehalt an Sterinverbindungen ca. 88 Gew.-% (56 Gew.-% Sitosterin, 28 Gew.-% Campesterin und 4 Gew.-% Stigmasterin), Sterinkohlenwasserstoffe und Cholesterin 4—6 Gew.-%, Triterpenalkohole, Testosteroide und andere steroidartige Bestandteile 4—6%.

Die Kultur der Stämme T 191 (DSM 1444) und T 191-1091 (ATCC 31 636) wird auf der Schüttelmaschine (Schüttelfrequenz 150 UpM) bei 30°C für 48 Stunden vorgezüchtet, anschließend 0,2 Gew.-% Emulgator und 0,5 Gew.-% des Pflanzensterins zugegeben und für weitere 120 Stunden bebrütet. Nach Abbruch der Kulturen werden Proben genommen, auf pH 2,0 eingestellt, 1 : 1 mit Essigester extrahiert und dünnschichtchromatographisch analysiert. Die jeweils erhaltenen Ausbeuten sind mit den vergleichbaren Werten des Beispiels 1 nahezu identisch.

## Beispiel 4

Der Defektmutantenstamm SC 372-837 (DSM 1990) ist in der Lage, erhöhte Mengen an $\Delta$1,4,17-BNC aus dem Pflanzensterin des Beispiels 3 zu bilden bzw. erlaubt es, die Dauer der Vorinkubation im Vergleich mit Beispiel 3 um die Hälfte zu verkürzen.

Die Stämme SC 372-837 werden in 500 ml Erlenmeyerkolben mit 100 ml Nährlösung folgender Zusammensetzung aerob gezüchtet: 0,5% Pepton, 0,8% Hefeextrakt, 0,4% Maiskleber, 0,3% Glucose, 0,05% Emulgator, 0,05% Pflanzensterin, pH 7,2. Die Kultur wird auf der Schüttelmaschine (Schüttelfrequenz 150 UpM) bei 30°C für 24 Stunden vorgezüchtet, anschließend 0,2% Emulgator und 0,5% Pflanzensterin zugegeben und für weitere 120 Stunden bebrütet. Nach Abbruch der Kulturen werden Proben genommen, auf pH 2,0 eingestellt, 1 : 1 mit Essigester extrahiert und dünnschichtchromatographisch analysiert.

| | |
|---|---|
| Ausbeute an $\Delta$1,4-BNC: | 49 mg/100 ml; |
| Ausbeute an $\Delta$1,4,17-BNC: | 15 mg/100 ml. |

## Beispiel 5

In den Beispielen 5 bis 7 wird als Mikroorganismenstamm DSM 1444 eingesetzt.

Die Defektmutanten wurden in 1,5 l-Kleinfermenter mit 1 l Nährlösung folgender Zusammensetzung aerob gezüchtet:

0,05% Pepton, 0,8% Hefeextrakt,
0,4% Maiskleber, 0,3% Glucose,
0,05% Emulgator, 0,1% Cholesterin bei pH 7,2.

Die Kultur wurde bei 30° C, 700 U/min und 1,0 VVM (VVM = Volumen Luft/Volumen Flüssigphase/Minute) für 24 Stunden vorinkubiert, anschließend 0,2% Emulgator und 0,4% Cholesterin zugegeben und für weitere 100 Stunden fermentiert.

Nach Abbruch der Fermentation wurden Proben entnommen, auf pH 2,0 eingestellt, 1 : 1 mit Essigester extrahiert und dünnschichtchromatographisch analysiert.

Die Ausbeuten sind nach Beispiel 12 zusammengefaßt.

## Beispiel 6

Die Defektmutanten wurden in 1,5 l-Kleinfermenter mit 1 l Nährlösung folgender Zusammensetzung aerob gezüchtet:

0,05% Pepton, 0,8% Hefeextrakt,
0,4% Maiskleber, 0,3% Glucose,
0,05% Emulgator, 0,1% Cholesterin bei pH 7,2.

Die Kultur wurde bei 30° C, 700 U/min und 0,5 VVM für 24 Stunden vorinkubiert, anschließend 0,2% Emulgator und 0,4% Cholesterin zugegeben und für weitere 100 Stunden fermentiert.

Nach Abbruch der Fermentation wurden Proben entnommen, auf pH 2,0 eingestellt, 1 : 1 mit Essigester extrahiert und dünnschichtchromatographisch analysiert.

Die Ausbeuten sind nach Beispiel 12 zusammengefaßt.

## Beispiel 7

Die Defektmutanten wurden in 1,5 l-Kleinfermenter mit 1 l Nährlösung folgender Zusammensetzung aerob gezüchtet:

0,05% Pepton, 0,8% Hefeextrakt,
0,4% Maiskleber, 0,3% Glucose,
0,05% Emulgator, 0,1% Cholesterin bei pH 7,2.

Die Kultur wurde bei 30° C, 700 U/min und 0,2 VVM für 24 Stunden vorinkubiert, anschließend 0,2% Emulgator und 0,4% Cholesterin zugegeben und für weitere 100 Stunden fermentiert.

Nach Abbruch der Fermentation wurden Proben entnommen, auf pH 2,0 eingestellt, 1 : 1 mit Essigester extrahiert und dünnschichtchromatographisch analysiert.

Die Ausbeuten sind nach Beispiel 12 zusammengefaßt.

## Beispiel 8

In den Beispielen 8 bis 12 werden als Defektmutanten SC 372-837 (DSM 1990) eingesetzt.

Die Defektmutanten wurden in 1,5 l-Kleinfermentern mit 1 l Nährlösung folgender Zusammensetzung aerob gezüchtet:

3% Tryptone Soya Broth, 0,1% $NH_4NO_3$, 0,6% Hefeextrakt,
0,05% myo Inosit, 0,15% Milchsäure,
0,15% Bernsteinsäure, 0,1% Emulgator bei pH 7,2.

Die Kultur wurde bei 30° C, 700 U/min und 1,0 VVM für 24 Stunden vorinkubiert, anschließend 0,1% Emulgator und 0,5% Pflanzensterin zugegeben und für weitere 100 Stunden fermentiert.

Nach Abbruch der Fermentation wurden Proben entnommen, auf pH 2,0 eingestellt, 1 : 1 mit Essigester extrahiert und dünnschichtchromatographisch analysiert.

Die Ausbeuten sind nach Beispiel 12 zusammengefaßt.

## Beispiel 9

Die Defektmutanten wurden in 1,5 l-Kleinfermentern mit 1 l Nährlösung folgender Zusammensetzung aerob gezüchtet:

3% Tryptone Soya Broth, 0,1% $NH_4NO_3$, 0,6% Hefeextrakt,
0,05% myo Inosit, 0,15% Milchsäure,
0,15% Bernsteinsäure, 0,1% Emulgator bei pH 7,2.

Die Kultur wurde bei 30° C, 700 U/min und 0,2 VVM für 24 Stunden vorinkubiert, anschließend 0,1% Emulgator (Tween 80) und 0,5% Pflanzensterin zugegeben und für weitere 100 Stunden fermentiert.
Nach Abbruch der Fermentation wurden Proben entnommen, auf pH 2,0 eingestellt, 1 : 1 mit Essigester extrahiert und dünnschichtchromatographisch analysiert.
Die Ausbeuten sind nach Beispiel 12 zusammengefaßt.

Beispiel 10

Die Defektmutanten wurden in 1,5 l-Kleinfermentern mit 1 l Nährlösung folgender Zusammensetzung aerob gezüchtet:

3% Tryptone Soya Broth, 0,1% $NH_4NO_3$, 0,6% Hefeextrakt,
0,05% myo Inosit, 0,15% Milchsäure,
0,15% Bernsteinsäure, 0,1% Emulgator bei pH 7,2.

Die Kultur wurde bei 30° C, 700 U/min und 0,5 VVM für 24 Stunden vorinkubiert, anschließend 0,1% Emulgator und 0,5% Pflanzensterin zugegeben und für weitere 24 Stunden fermentiert.
Nach Abbruch der Fermentation wurden Proben entnommen, auf pH 2,0 eingestellt, 1 : 1 mit Essigester extrahiert und dünnschichtchromatographisch analysiert.
Die Ausbeuten sind nach Beispiel 12 zusammengefaßt.

Beispiel 11

Die Defektmutanten wurden in 1,5 l-Kleinfermentern mit 1 l Nährlösung folgender Zusammensetzung aerob gezüchtet:

3% Tryptone Soya Broth, 0,1% $NH_4NO_3$, 0,6% Hefeextrakt,
0,05% myo Inosit, 0,15% Milchsäure,
0,15% Bernsteinsäure, 0,1% Emulgator bei pH 7,2.

Die Kultur wurde bei 30° C, 700 U/min und 1 VVM für 24 Stunden vorinkubiert, anschließend 0,1% Emulgator, 3% Dimethylformamid und 0,5% Pflanzensterin zugegeben und für weitere 24 Stunden fermentiert.
Nach Abbruch der Fermentation wurden Proben entnommen, auf pH 2,0 eingestellt, 1 : 1 mit Essigsäure extrahiert und dünnschichtchromatographisch analysiert.
Die Ausbeuten sind nach Beispiel 12 zusammengefaßt.

Beispiel 12

Die Defektmutanten wurden in 1,5 l-Kleinfermentern mit 1 l Nährlösung folgender Zusammensetzung aerob gezüchtet:

3% Tryptone Soya Broth, 0,1% $NH_4NO_3$, 0,6% Hefeextrakt,
0,05% myo Inosit, 0,15% Milchsäure,
0,15% Bernsteinsäure, 0,1% Emulgator bei pH 7,2.

Die Kultur wurde bei 30° C, 700 U/min und 1 VVM für 24 Stunden vorinkubiert, anschließend 0,1% Emulgator, 3% Dimethylformamid und 0,5% Pflanzensterin zugegeben und für weitere 100 Stunden fermentiert.
Nach Abbruch der Fermentation wurden Proben entnommen, auf pH 2,0 eingestellt, 1 : 1 mit Essigsäure extrahiert und dünnschichtchromatographisch analysiert.
Die Ausbeuten sind nach Beispiel 12 zusammengefaßt.

| Interne Nr. | Hinterlegungs-Nr. | Beispiel | $\Delta$1,4-BNC mg/100 ml | $\Delta$1,4,17-BNC mg/100 ml | $pO_2$ % |
|---|---|---|---|---|---|
| T 191 | DSM 1444 | 5 | 350 | 40 | 100 |
| | | 6 | 200 | 75 | 70 |
| | | 7 | 150 | 65 | 30 |
| SC 372-837 | DSM 1990 | 8 | 66 | 10 | 100 |
| | | 9 | 60 | 25 | 30 |
| | | 10 | 37 | 35 | 65 |
| | | 11 | 71 | 36 | 100 |
| | | 12 | 95 | 46 | 100 |

**Patentansprüche**

1. Neue $\Delta$1,4,17-BNC-Verbindungen der allgemeinen Formel I

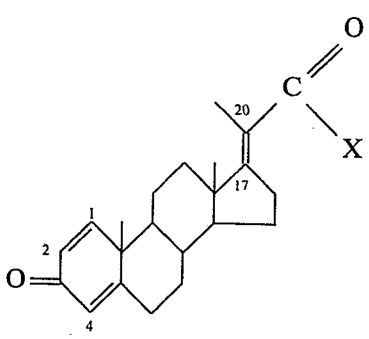

(I)

in der X einen der Reste OH oder $OCH_3$ bedeutet.

2. Verfahren zur Herstellung von 20-Carboxy-pregna-1,4,17(20)-trien-3-on ($\Delta$1,4,17-BNC) durch mikrobiellen Seitenkettenabbau an 17-C-Seitenketten-Steroidsubstraten in einem wäßrigen Nährmedium unter aeroben Bedingungen mit Mikroorganismen-Defektmutanten, die auch in Abwesenheit von den Steroidringabbau und/oder das Wachstum hemmenden Inhibitoren Steroidverbindungen mit dem 17-C-$\alpha$-Propionsäurerest liefern und die gemäß den europäischen Patentanmeldungen 0 004 913 und 0 015 308 erhältlich sind, dadurch gekennzeichnet, daß man die Mikroorganismen-Defektmutanten bei gedrosselter Luftzufuhr unter Anreicherung der $\Delta$1,4,17-BNC züchtet und diese aus dem Reaktionsprodukt abtrennt.

3. Verfahren nach Anspruch 2, dadurch gekennzeichnet, daß man mit einer Sättigungskonzentration des Sauerstoffs in der Fermenter-Flüssigphase von höchstens 90%, vorzugsweise mit entsprechenden Sättigungskonzentrationen im Bereich von 30 bis 70% arbeitet.

4. Verfahren nach Ansprüchen 2 und 3, dadurch gekennzeichnet, daß man mit derart gedrosselter Luftzufuhr arbeitet, daß eine Senkung der Gesamtausbeute an BNC-Verbindungen um wenigstens 5 Gewichtsprozent, vorzugsweise um wenigstens 10 Gewichtsprozent — verglichen mit identischer Arbeitsweise aber unbeschränktem Luftzutritt — eintritt.

5. Verfahren nach Ansprüchen 2 bis 4, dadurch gekennzeichnet, daß man unter Mitverwendung von wenigstens teilweise wassermischbaren organischen Lösungsmitteln für das eingesetzte Steroidsubstrat arbeitet.

6. Verfahren nach Anspruch 5, dadurch gekennzeichnet, daß man unbeschränkt wassermischbare Lösungsmittel einsetzt und dabei das Steroidsubstrat im Lösungsmittel gelöst in den Fermenter einträgt.

7. Verfahren nach Ansprüchen 2 bis 6, dadurch gekennzeichnet, daß man als Ausgangsmaterial Steroidverbindungen mit gesättigten und/oder ungesättigten 17-C-Seitenketten mit vorzugsweise bis zu 10 C-Atomen einsetzt, wobei insbesondere Sterinverbindungen tierischen und/oder pflanzlichen Ursprungs als Ausgangsmaterial eingesetzt werden.

8. Verfahren nach Ansprüchen 2 bis 7, dadurch gekennzeichnet, daß man mit Defektmutanten von DSM 1444 — insbesondere ATCC 31 636 — oder mit DSM 1990 arbeitet.

## Claims

1. New $\Delta$1,4,17-BNC-compounds corresponding to the following general formula

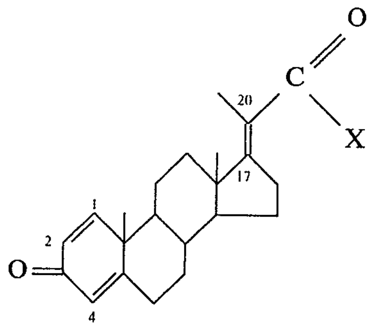

(I)

in which X is one of the residues OH or $OCH_3$.

2. A process for producing 20-carboxy-pregna-1,4,17(20)-trien-3-one ($\Delta$1,4,17-BNC) by microbial side chain degradation on 17-C-side chain steroid substrates in an aqueous nutrient medium under aerobic conditions using deficient microorganism mutants which yield steroid compounds containing the 17-C-$\alpha$-propionic acid residue, even in the absence of inhibitors which inhibit degradation of the steroid ring and/or growth and which are obtainable in accordance with European Patent Applications 0 004 913 and 0 015 308, characterized in that the deficient microorganism mutants are grown in a reduced air supply with enrichment of the $\Delta$-1,4,17-BNC and the $\Delta$1,4,17-BNC is separated off from the reaction product.

3. A process as claimed in Claim 2, characterized in that it is carried out at a saturation concentration of the oxygen in the fermenter liquid phase of at most 90% and preferably at corresponding saturation concentrations of from 30 to 70%.

4. A process as claimed in Claims 2 and 3, characterized in that the air supply is reduced to such an extent that the total yield of BNC-compounds is reduced by at least 5% by weight and preferably by at least 10% by weight compared with the same procedure but an unlimited air supply.

5. A process as claimed in Claims 2 to 4, characterized in that it is carried out using at least partly water-miscible organic solvents for the steroid substrate used.

6. A process as claimed in Claim 5, characterized in that solvents indefinitely miscible with water are used and the steroid substrate is introduced into the fermenter in solution in the solvent.

7. A process as claimed in Claims 2 to 6, characterized in that steroid compounds with saturated and/or unsaturated 17-C-side chains referably containing up to 10 C-atoms and, more particularly, sterol compounds of animal and/or vegetable origin are used as starting material.

8. A process as claimed in Claims 2 to 7, characterized in that deficient mutants of DSM 1444, more particularly ATCC 31 636, or DSM 1990 are used.

## Revendications

1. Nouveaux composés en $\Delta$1,4,17-BNC de formule générale I

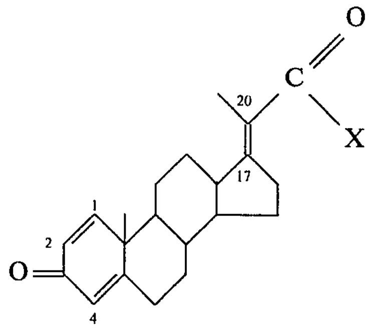

(I)

dans laquelle X représente un groupe OH ou $OCH_3$.

2. Procédé de préparation de la 20-carboxy-prégna-1,4,17(20)-triène-3-one ($\Delta$1,4,17-BNC) par dégradation microbienne des chaînes latérales de substrats stéroïdiques à chaînes latérales en C 17 dans un milieu nutritif aqueux dans des conditions aérobies par des mutants déficients bloqués de microorganismes qui donnent des composés stéroïdiques portant le reste d'acide alpha-propionique en C 17 même en l'absence d'inhibiteurs de la dégradation des cycles des stéroïdes et/ou de la croissance et qui peuvent être obtenus comme décrit dans les demandes de brevets européens publiées sous n°

0 004 913 et 0 015 308, caractérisé en ce que l'on cultive les mutants déficients bloqués de microorganismes avec alimentation étranglée en air, avec accumulation de la Δ1,4,17-BNC qu'on sépare du produit de réaction.

3. Procédé selon la revendication 2, caractérisé en ce que l'on opère à une concentration de saturation de l'oxygène dans la phase liquide du fermenteur qui est au maximum de 90%, de préférence à de telles concentrations de saturation qui se situent dans l'intervalle de 30 à 70%.

4. Procédé selon les revendications 2 et 3, caractérisé en ce que l'on opère avec alimentation étranglée en air telle qu'il y ait une diminution du rendement global en composés du type BNC d'au moins 5% en poids, de préférence d'au moins 10% en poids, par rapport à des opérations identiques avec alimentation non limitée en air.

5. Procédé selon les revendications 2 à 4, caractérisé en ce que l'on opère en utilisant conjointement des solvants organiques miscibles à l'eau en partie au moins pour le substrat stéroïdique mis en œuvre.

6. Procédé selon la revendication 5, caractérisé en ce que l'on utilise des solvants entièrement miscibles à l'eau et en ce que l'on introduit le substrat stéroïdique dans le fermenteur à l'état de solution dans le solvant.

7. Procédé selon les revendications 2 à 6, caractérisé en ce que l'on utilise en tant que produits de départ des composés stéroïdiques portant sur le C 17 des chaînes latérales saturées et/ou insaturées contenant de préférence jusqu'à 10 atomes de carbone, et en particulier des stérols et dérivés d'origine animale et/ou végétale.

8. Procédé selon les revendications 2 à 7, caractérisé en ce que l'on opère avec des mutants déficients bloqués de DSM 1444 — en particulier ATCC 31 636 — ou avec DSM 1990.